# EUROPEAN PATENT APPLICATION

(11) **EP 3 075 856 A1**
(43) Date of publication of application: **05.10.2016**
(21) Application number: 14865169.8
(22) Date of filing: 29.10.2014
(51) Int. Cl.: C12N 15/54, C12N 15/31, C12N 15/11, C12N 9/10, C12P 41/00, C07K 14/195

(54) **OMEGA-TRANSAMINASE OF R CONFIGURATION AND USE THEREOF**

(30) Priority: 26.11.2013 CN 201310610701
(71) Applicant: Asymchem Laboratories (Tianjin) Co., Ltd, Tianjin 300457 (CN); Asymchem Life Science (Tianjin) Co., Ltd, Tianjin 300457 (CN); Tianjin Asymchem Pharmaceutical Co., Ltd, Tianjin 300000 (CN); Asymchem Laboratories (Fuxin) Co., Ltd, Fuxin, Liaoning 123000 (CN); Jilin Asymchem Laboratories Co., Ltd, Dunhua, Jilin 133700 (CN)
(72) Inventor: HONG, Hao, Tianjin 300457 (CN); GAO, Feng, Tianjin 300457 (CN); LI, Yanjun, Tianjin 300457 (CN); ZHANG, Yan, Tianjin 300457 (CN); LI, Shaohe, Tianjin 300457 (CN)
(74) Representative: Zimmermann, Tankred Klaus
(86) International application number: PCT/CN2014/089845
(87) International publication number: WO 2015/078258

(57) **Abstract**

An omega-transaminase of R-configuration is provided. The omega-transaminase of R-configuration has the amino acid sequence as shown in SEQ ID NO: 2, or has at least 80% identity to the amino acid sequence as shown in SEQ ID NO: 2, or has the amino acid sequence of proteins which have substituted, deleted or added one or more amino acids and have the activity of an omega-transaminase with a high stereoselectivity R-configuration catalytic activity; and does not have the amino acid sequence encoded by the nucleotide sequence as shown in SEQ ID NO: 4. The high stereoselectivity refers to the content of one of the stereoisomers being at least about 1.1 times that of the other. A use of the omega-transaminase of R-configuration is provided, which can be used for highly efficient synthesis of a chiral amine of R-configuration with a relatively high chiral purity, and is therefore suitable for industrial production of chiral amines.

Sequenceing List

<110>  ASYMCHEM LABORATORIES (TIANJIN) CO.,LTD
       ASYMCHEM LIFE SCIENCE (TIANJIN) CO.,LTD
       TIANJIN ASYMCHEM PHARMACEUTICAL CO.,LTD
       ASYMCHEM LABORATORIES (FUXIN) CO.,LTD
       JILIN ASYMCHEM LABORATORIES CO.,LTD

<120>  Omega-transaminase of R configuration and uses thereof

<130>  PN46048KLY

<160>  8

<170>  PatentIn version 3.2

<210>  SEQ ID NO:1
<211>  999
<212>  DNA
<213>  Artificial sequence

<220>
<223> modified transaminase gene from Arthrobacter sp.

<400>  1
atggcgttta ctgtagaaag cccggcgagt atggataagg tcttcgccgg ctatgctgcc      60

cgccaggcta tcctcgaaag tacggagacg acgaacccat tcgccaaagg tattgcatgg     120

gtcgaaggtg agttagttcc gctggccgaa gcacgtatcc cgattctcga tcagggcttt     180

tactccagcg acgcgaccta taccaccttc catgtctgga acggcaacgc gttccgtctg     240

ggtgaccaca tcgaacgtct gttcagcaat gcggagagca tccgtctgat tccgccgctg     300

actcaggacg aagtgaaaga aatcgccctg gaactggtgg cgaaaacgga actgcgtgaa     360

gcaatggtca ccgtcaccat cactcgtggt tactcctcta ccccattcga acgcgatatc     420

actaaacacc gcccgcaggt ttacatgtct gcgtgtccgt accagtggat cgtaccgttt     480

gaccgtatcc gtgatggcgt gcacctgatg gtagctcagt ctgttcgccg cactccgcgt     540

agcagcatcg atccgcaagt taagaacttc caatggggcg atctgatccg tgcgatccag     600

gagacccacg accgtggctt cgaactgcct ctgctgctgg attgcgataa cctgctggct     660

gaaggcccgg gctttaacgt cgtagttatt aaagacggcg ttgttcgttc tccgggtcgc     720

gctgccctgc cgggtattac ccgcaaaacc gttctggaaa tcgctgagtc cctgggtcat     780

gaggctatcc tggctgacat tacgccagcc gaactgtacg atgctgatga agtactgggt     840

tgctctactg gtggtggcgt ttggccgttc gtatctgtgg acggtaattc catctccgat     900

ggtgttccgg gccctgtgac ccagtccatt attcgccgtt actgggaact gaacgtggag     960

ccgtcttctc tgctgactct tgttgagtac gcactcgag                            999

<210>  SEQ ID NO:2

<211>  333
<212>  PRT
<213>  Artificial sequence

<220>
<223> protein of the modified transaminase gene from Arthrobacter sp.

<400>  2

Met Ala Phe Thr Val Glu Ser Pro Ala Ser Met Asp Lys Val Phe Ala
1               5                   10                  15

Gly Tyr Ala Ala Arg Gln Ala Ile Leu Glu Ser Thr Glu Thr Thr Asn
            20                  25                  30

Pro Phe Ala Lys Gly Ile Ala Trp Val Glu Gly Glu Leu Val Pro Leu
        35                  40                  45

Ala Glu Ala Arg Ile Pro Ile Leu Asp Gln Gly Phe Tyr Ser Ser Asp
    50                  55                  60

Ala Thr Tyr Thr Thr Phe His Val Trp Asn Gly Asn Ala Phe Arg Leu
65                  70                  75                  80

Gly Asp His Ile Glu Arg Leu Phe Ser Asn Ala Glu Ser Ile Arg Leu
                85                  90                  95

Ile Pro Pro Leu Thr Gln Asp Glu Val Lys Glu Ile Ala Leu Glu Leu
            100                 105                 110

Val Ala Lys Thr Glu Leu Arg Glu Ala Met Val Thr Val Thr Ile Thr
        115                 120                 125

Arg Gly Tyr Ser Ser Thr Pro Phe Glu Arg Asp Ile Thr Lys His Arg
    130                 135                 140

Pro Gln Val Tyr Met Ser Ala Cys Pro Tyr Gln Trp Ile Val Pro Phe
145                 150                 155                 160

Asp Arg Ile Arg Asp Gly Val His Leu Met Val Ala Gln Ser Val Arg
                165                 170                 175

Arg Thr Pro Arg Ser Ser Ile Asp Pro Gln Val Lys Asn Phe Gln Trp
            180                 185                 190

Gly Asp Leu Ile Arg Ala Ile Gln Glu Thr His Asp Arg Gly Phe Glu
        195                 200                 205

Leu Pro Leu Leu Leu Asp Cys Asp Asn Leu Leu Ala Glu Gly Pro Gly
    210                 215                 220

Phe Asn Val Val Val Ile Lys Asp Gly Val Val Arg Ser Pro Gly Arg
225                 230                 235                 240

Ala Ala Leu Pro Gly Ile Thr Arg Lys Thr Val Leu Glu Ile Ala Glu
                245                 250                 255

Ser Leu Gly His Glu Ala Ile Leu Ala Asp Ile Thr Pro Ala Glu Leu
            260                 265                 270

Tyr Asp Ala Asp Glu Val Leu Gly Cys Ser Thr Gly Gly Gly Val Trp
        275                 280                 285

Pro Phe Val Ser Val Asp Gly Asn Ser Ile Ser Asp Gly Val Pro Gly
    290                 295                 300

Pro Val Thr Gln Ser Ile Ile Arg Arg Tyr Trp Glu Leu Asn Val Glu
305                 310                 315                 320

Pro Ser Ser Leu Leu Thr Leu Val Glu Tyr Ala Leu Glu
                325                 330

<210>  SEQ ID NO:3
<211>  198
<212>  DNA
<213>  Artificial sequence

<220>
<223> a sequence disclosed in NCBI is used as primer for replaction
of the recombinant vector pET22B-taA.

<400>  3
atggcgttta ctgtagaaag cccggcgagt atggataagg tcttcgccgg ctatgctgcc      60

cgccaggcta tcctcgaaag tacggagacg acgaacccat tcgccaaagg tattgcatgg     120

gtcgaaggtg agttagttcc gctggccgaa gcacgtatcc cgattctcga tcagggcttt     180

tactccagcg acgcgacc                                                   198

<210>  SEQ ID NO:4
<211>  999
<212>  DNA
<213>  Arthrobacter sp.

<400>  4
atggcattta gcgcagatac accggaaatt gtttataccc atgataccgg tctggattat      60

attacctata gcgattatga actggatccg gcaaatccgc tggcaggcgg tgcagcatgg     120

attgaaggtg catttgttcc tccgagcgaa gcacgtatta gcatttttga tcagggcttt     180

tataccagtg atgcaaccta taccaccttt catgtgtgga atggtaatgc atttcgtctg     240

ggtgatcata ttgaacgtct gtttagcaat gccgaaagca ttcgtctgat tcctccgctg 300

acccaggatg aagttaaaga aattgcactg gaactggttg caaaaaccga actgcgtgaa 360

gcaatggtta ccgttaccat tacccgtggt tatagcagca ccccgtttga acgtgatatt 420

accaaacatc gtccgcaggt ttatatgagc gcatgtccgt atcagtggat tgttccgttt 480

gatcgtattc gtgatggtgt tcatctgatg gttgcacaga gcgttcgtcg tacaccgcgt 540

agcagcattg atccgcaggt taaaaatttt cagtggggtg atctgattcg tgcaattcag 600

gaaacccacg atcgcggttt tgaactgccg ctgctgctgg attgtgataa tctgctggcc 660

gaaggtccgg gttttaatgt tgttgttatt aaagatggcg tggttcgtag tccgggtcgt 720

gcagcactgc ctggtattac ccgtaaaacc gttctggaaa ttgcagaaag cctgggtcat 780

gaagcaattc tggcagatat tacaccggca gaactgtatg atgcagatga agttctgggt 840

tgtagcaccg gtggtggtgt ttggccgttt gttagcgttg atggtaatag cattagtgat 900

ggcgttccgg gtccggttac ccagagcatt attcgtcgtt attgggaact gaatgttgaa 960

ccgagcagcc tgctgacacc ggttcagtat gcactcgag 999

<210> SEQ ID NO:5
<211> 333
<212> PRT
<213> Arthrobacter sp.

<400> 5

Met Ala Phe Ser Ala Asp Thr Pro Glu Ile Val Tyr Thr His Asp Thr
1               5                   10                  15

Gly Leu Asp Tyr Ile Thr Tyr Ser Asp Tyr Glu Leu Asp Pro Ala Asn
            20                  25                  30

Pro Leu Ala Gly Gly Ala Ala Trp Ile Glu Gly Ala Phe Val Pro Pro
        35                  40                  45

Ser Glu Ala Arg Ile Ser Ile Phe Asp Gln Gly Phe Tyr Thr Ser Asp
    50                  55                  60

Ala Thr Tyr Thr Thr Phe His Val Trp Asn Gly Asn Ala Phe Arg Leu
65                  70                  75                  80

Gly Asp His Ile Glu Arg Leu Phe Ser Asn Ala Glu Ser Ile Arg Leu
                85                  90                  95

Ile Pro Pro Leu Thr Gln Asp Glu Val Lys Glu Ile Ala Leu Glu Leu
            100                 105                 110

Val Ala Lys Thr Glu Leu Arg Glu Ala Met Val Thr Val Thr Ile Thr
        115                 120                 125

Arg Gly Tyr Ser Ser Thr Pro Phe Glu Arg Asp Ile Thr Lys His Arg
    130                 135                 140

Pro Gln Val Tyr Met Ser Ala Cys Pro Tyr Gln Trp Ile Val Pro Phe
145                 150                 155                 160

Asp Arg Ile Arg Asp Gly Val His Leu Met Val Ala Gln Ser Val Arg
                165                 170                 175

Arg Thr Pro Arg Ser Ser Ile Asp Pro Gln Val Lys Asn Phe Gln Trp
            180                 185                 190

Gly Asp Leu Ile Arg Ala Ile Gln Glu Thr His Asp Arg Gly Phe Glu
        195                 200                 205

Leu Pro Leu Leu Leu Asp Cys Asp Asn Leu Leu Ala Glu Gly Pro Gly
    210                 215                 220

Phe Asn Val Val Val Ile Lys Asp Gly Val Val Arg Ser Pro Gly Arg
225                 230                 235                 240

Ala Ala Leu Pro Gly Ile Thr Arg Lys Thr Val Leu Glu Ile Ala Glu
                245                 250                 255

Ser Leu Gly His Glu Ala Ile Leu Ala Asp Ile Thr Pro Ala Glu Leu
            260                 265                 270

Tyr Asp Ala Asp Glu Val Leu Gly Cys Ser Thr Gly Gly Gly Val Trp
        275                 280                 285

Pro Phe Val Ser Val Asp Gly Asn Ser Ile Ser Asp Gly Val Pro Gly
    290                 295                 300

Pro Val Thr Gln Ser Ile Ile Arg Arg Tyr Trp Glu Leu Asn Val Glu
305                 310                 315                 320

Pro Ser Ser Leu Leu Thr Pro Val Gln Tyr Ala Leu Glu
                325                 330

<210>  SEQ ID NO:6
<211>  27
<212>  DNA
<213>  Artificial sequence

<220>

<223>the primer of the sense strand

<400> 6
cgcaagttaa gaacttccaa tggggcg 27


<210> SEQ ID NO:7
<211> 32
<212> DNA
<213> Artificial sequence

<220>
<223>the primer of the antisense strand

<400> 7
ccattggaag ttcttaactt gcggatcgat gc 32


<210> SEQ ID NO:8
<211> 333
<212> PRT
<213> Artificial sequence

<220>
<223> Ile to Val substitution at site of 199 of SEQ ID NO:2

<400> 8

Met Ala Phe Thr Val Glu Ser Pro Ala Ser Met Asp Lys Val Phe Ala
1               5                   10                  15


Gly Tyr Ala Ala Arg Gln Ala Ile Leu Glu Ser Thr Glu Thr Thr Asn
            20                  25                  30


Pro Phe Ala Lys Gly Ile Ala Trp Val Glu Gly Glu Leu Val Pro Leu
        35                  40                  45


Ala Glu Ala Arg Ile Pro Ile Leu Asp Gln Gly Phe Tyr Ser Ser Asp
    50                  55                  60


Ala Thr Tyr Thr Thr Phe His Val Trp Asn Gly Asn Ala Phe Arg Leu
65                  70                  75                  80


Gly Asp His Ile Glu Arg Leu Phe Ser Asn Ala Glu Ser Ile Arg Leu
                85                  90                  95


Ile Pro Pro Leu Thr Gln Asp Glu Val Lys Glu Ile Ala Leu Glu Leu
            100                 105                 110


Val Ala Lys Thr Glu Leu Arg Glu Ala Met Val Thr Val Thr Ile Thr
        115                 120                 125


Arg Gly Tyr Ser Ser Thr Pro Phe Glu Arg Asp Ile Thr Lys His Arg
    130                 135                 140

Pro Gln Val Tyr Met Ser Ala Cys Pro Tyr Gln Trp Ile Val Pro Phe
145                 150                 155                 160

Asp Arg Ile Arg Asp Gly Val His Leu Met Val Ala Gln Ser Val Arg
                165                 170                 175

Arg Thr Pro Arg Ser Ser Ile Asp Pro Gln Val Lys Asn Phe Gln Trp
            180                 185                 190

Gly Asp Leu Ile Arg Ala Val Gln Glu Thr His Asp Arg Gly Phe Glu
        195                 200                 205

Leu Pro Leu Leu Leu Asp Cys Asp Asn Leu Leu Ala Glu Gly Pro Gly
    210                 215                 220

Phe Asn Val Val Val Ile Lys Asp Gly Val Val Arg Ser Pro Gly Arg
225                 230                 235                 240

Ala Ala Leu Pro Gly Ile Thr Arg Lys Thr Val Leu Glu Ile Ala Glu
                245                 250                 255

Ser Leu Gly His Glu Ala Ile Leu Ala Asp Ile Thr Pro Ala Glu Leu
            260                 265                 270

Tyr Asp Ala Asp Glu Val Leu Gly Cys Ser Thr Gly Gly Gly Val Trp
        275                 280                 285

Pro Phe Val Ser Val Asp Gly Asn Ser Ile Ser Asp Gly Val Pro Gly
    290                 295                 300

Pro Val Thr Gln Ser Ile Ile Arg Arg Tyr Trp Glu Leu Asn Val Glu
305                 310                 315                 320

Pro Ser Ser Leu Leu Thr Leu Val Glu Tyr Ala Leu Glu
                325                 330

## Description

### Technical field

The present invention relates to the field of synthesis of chiral compounds, and particularly to an omega-transaminase of R-configuration and uses thereof.

### Background

Chiral amines are ubiquitous in nature. They are common structural units in many important bioactive molecules, both synthetic and natural in origin. Chiral amines are a common structural motif in many drugs. Many chiral amines are also important chiral auxiliaries and chiral selectors. Therefore, the preparation of chiral amines is of significant economic importance.

At present, chiral amines are mainly prepared by means of chemical reduction, and amines with optical activity are prepared by using prochiral ketones. Catalyzed by Pd/C and quinine, a prochiral ketone reacts with formic acid and an ammonia or an organic primary amine to generate a chiral amine. Other researchers obtained a chiral amine through asymmetric amination and reduction of a prochiral ketone using a ruthenium complex as a catalyst (Renat Kadyrov et al. Highly Enantioselective Hydrogen-Transfer Reductive Amination: Catalytic Asymmetric Synthesis of Primary Amines. Angewandte Chemie International Edition. 2003, 42 (44), Page 5472 to Page 5474). The metal catalyst in such a reaction is a very critical factor and demands strict requirements on the metal catalyst. Also, it is necessary to carry out the reaction at high temperature and there are high requirements on operation devices. Additionally, the metal catalyst is expensive and an environmental pollutant (Ohkuma T et al. Trans-RuH (eta1-BH4) (binap) (1,2-diamine): a catalyst for asymmetric hydrogenation of simple ketones under base-free conditions. Journal of the American Chemical Society.2002, 124(23), Page 6508 to Page 6509).

An aminotransferase, also known as a transaminase, may catalyze an exchange process between amino and carbonyl groups of alpha-amino acids. An omega-transaminase is a transaminase capable of catalysing a transamination reaction using substrates other than alpha-amino acid. Omega-transaminase may effectively produce a chiral amine through stereoselective transamination using a variety of ketones as raw materials. The omega-transaminase has attracted more and more attention by researchers because of its use of relatively cheap substrates and its ability to produce highly pure products. It is expected that the potential of omega-transaminase can be fully applied toward the industrial production of chiral amines. However, there is still a need for further research and invention of this class of enzyme.

There is a demand for an omega-transaminase with high catalytic activity and stereoselectivity toward the R-configuration so that demand for chiral amine can be satisfied.

### Summary

The present invention aims to provide an omega-transaminase of R-configuration and the uses thereof so as to overcome the deficiencies of omega-transaminase found in the prior art which are inadequate at satisfying demand for industrial production of chiral amines.

An omega-transaminase of R-configuration or a modified compound, functional equivalent, functional fragment or variant thereof is provided according to an aspect of the present invention so as to achieve the purpose above. The amino sequence of the omega-transaminase of R-configuration include a sequence selected from one of the following sequences: a) an amino acid sequence as shown in SEQ ID NO: 2; b) an amino acid sequence with at least 80% identity to the amino acid sequences as shown in SEQ ID NO: 2 and having the activity of an omega-transaminase with high stereoselective R-configuration catalytic activity, wherein the amino acid sequence is not an amino acid sequence encoded by a nucleotide sequence as shown in SEQ ID NO: 4; c) a protein which is derived from SEQ ID NO: 2 by subjecting the amino acid sequence as shown in SEQ ID NO: 2 to substitution, deletion or addition one or more amino acids, and having the activity of an omega-transaminase with high stereoselective R-configuration catalytic activity, wherein the amino acid sequence is not an amino acid sequence encoded by the nucleotide sequence as shown in SEQ ID NO: 4, wherein the high stereoselectivity refers to the content of one of the stereoisomers being at least about 1.1 times that of the other.

Further, the amino acid sequence of the omega-transaminase of R-configuration is an amino acid sequence acquired by substituting leucine at the 199^{th} site in the amino acid sequence as shown in SEQ ID NO: 2 by valine.

A nucleotide is provided according to another aspect of the present invention. The nucleotide encodes the omega-transaminase of R-configuration or the modified compound, functional equivalent, functional fragment or variant thereof.

Further, the sequence of the nucleotide include a sequence selected from one of the following sequences: a) a nucleotide sequence as shown in SEQ ID NO: 1; b) a nucleotide sequence with at least 80% identity to the nucleotide sequence as shown in SEQ ID NO: 1 and encoding an omega-transaminase with high stereoselective R-configuration catalytic activity, wherein the nucleotide sequence is not a nucleotide sequence as shown in SEQ ID NO: 4; c) a nucleotide sequence capable of hybridizing with the nucleotide sequence as shown in SEQ ID NO: 1 under highly stringent conditions and encoding an omega-transaminase with high stereoselective R-configuration catalytic activity, wherein the nucleotide sequence is not the nucleotide sequence as shown in SEQ ID NO: 4, wherein the high stereoselectivity refers to the content of one of the stereoisomers being at least about 1.1 times that of the other.

A recombinant vector is provided according to another aspect of the present invention. Any foregoing nucleotide is effectively connected in the recombinant vector.

Further, the recombinant vector is pET22b-taC.

A host cell is provided according to another aspect of the present invention. Any foregoing recombinant vector is transformed or transfected into the host cell.

A method for synthesizing a chiral amine is provided according to another aspect of the present invention. The method comprises the following steps: making a ketone compound, the omega-transaminase of R-configuration or the modified compound, functional equivalent, functional fragment or variant thereof above mentioned, pyridoxal phosphate, and an amino donor to react in a reaction system so as to obtain the chiral amine.

Further, the ketone compound is 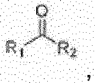 where R₁ and R₂ are independently C₁ to C₈ alkyl, C₅ to C₁₀ naphthenic base, C₅ to C₁₀ aryl or C₅ to C₁₀ heteroaryl; or R₁ and R₂ form a C₅ to C₁₀ heterocyclic radical, a C₅ to C₁₀ carbocyclic radical or C₅ to C₁₀ heteroaryl with a carbon on a carbonyl group; heteroatoms in the C₅ to C₁₀ heterocyclic radical and C₅ to C₁₀ heteroaryl are independently selected from at least one of nitrogen, oxygen and sulfur; the aryl in the C₅ to C₁₀ aryl, the heteroaryl in the C₅ to C₁₀ heteroaryl, the carbocyclic radical in the C₅ to C₁₀ carbocyclic radical or the heterocyclic radical in the C₅ to C₁₀ heterocyclic radical is independently unsubstituted or is substituted by at least one radical of halogen, alkoxy or alkyl; preferably, the ketone compound 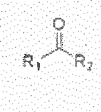 is selected from 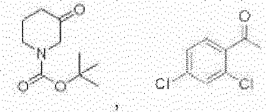 or

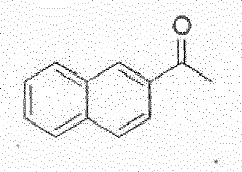

Further, the reaction system further comprises a dissolution promoter, and the dissolution promoter is dimethyl sulfoxide or polyethylene glycol, and the polyethylene glycol is preferably PEG-400.

Further, the C1 to C8 alkyl is C1 to C8 linear alkyl, the C5 to C10 heteroaryl is a pyridine group, the alkoxy is C1 to C6 alkoxy, the heterocyclic radical in the C5 to C10 heterocyclic radical is piperidine, a substituent on the aryl in the C5 to C10 aryl, the heteroaryl in the C5 to C10 heteroaryl, the carbocyclic radical in the C5 to C10 carbocyclic radical or the heterocyclic radical in the C5 to C10 heterocyclic radical is independently C1 to C6 linear alkyl or C1 to C6 alkoxy, and the amino donor is isopropylamine or D-alanine.

By means of the technical solutions of the present invention, an omega-transaminase of R-configuration having a high stereoselectivity, or a modified compound, functional equivalent, functional fragment or variant thereof may be used for highly efficient synthesis of a chiral amine of R-configuration with a relatively high chiral purity, and is therefore suitable for industrial production of chiral amines.

### Brief description of the drawings

The accompanying drawings of the specification, which constitute a part of the invention, are used for providing further understanding to the present invention. The exemplary embodiments of the present invention and illustration thereof are used for explaining the present invention, instead of constituting improper limitation to the present invention. In the accompanying drawings:

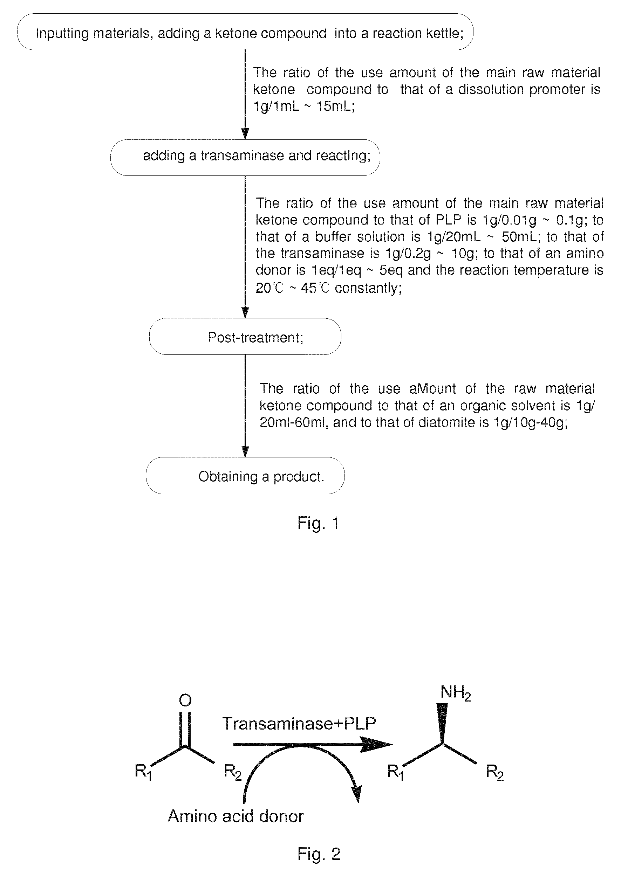

Fig. 1 shows a flowchart of a chemical reaction of a use of an omega-transaminase of R-configuration according to the present invention in synthesis of a chiral amine;
Fig. 2 shows an equation of a chemical reaction of a use of an omega-transaminase of R-configuration according to the present invention in synthesis of a chiral amine;
Fig. 3 shows an identification result of enzyme digestion in the first embodiment of the present invention;
Fig. 4 shows a sequencing result of a mutant gene having been subjected to a Polymerase Chain Reaction (PCR) in the first embodiment of the present invention; and
Fig. 5 shows a protein expressed by a recombinant plasmid pET22b-taB in the first embodiment of the present invention.

### Detailed description of the embodiments

It needs to be noted that the embodiments in the invention and the characteristics in the embodiments may be combined with each other if there is no conflict. The present invention will be expounded hereinafter with reference to the accompanying drawings and in conjunction with the embodiments.

### Term explanation

The term "optional/random" or "optionally/randomly" means that an event or a situation in description thereinafter may happen or may not happen. The description includes that the event or the situation happens or does not happen. For example, "optionally substituted alkyl" refers to "unsubstituted alkyl" (alkyl that has not been substituted by a substituent) or "substituted alkyl" (alkyl that has been substituted by a substituent), as defined hereinafter.

The C1 to Cn used herein includes C1 to C2, C1 to C3, ......C1 to Cn. For example, the "C1 to C4" radical means that the part has 1 to 4 carbon atoms, that is, the radical includes 1 carbon atom, 2 carbon atoms, 3 carbon atoms or 4 carbon atoms.

The term "alkyl" used separately or in combination herein refers to optionally substituted linear chain or optionally substituted branched chain aliphatic hydrocarbons. The "alkyl" herein may preferably have 1 to 20 carbon atoms, e.g. 1 to 10 carbon atoms, 1 to 8 carbon atoms, 1 to 6 carbon atoms, 1 to 4 carbon atoms or 1 to 3 carbon atoms. The term "alkoxy" used separately or in combination herein refers to an alkyl ether group (O-alkyl). Nonrestrictive embodiments of alkoxy include: methoxy, ethoxy, n-propoxy, isopropoxy, n-butoxy, isobutoxy, sec-butoxy, tert-butoxy and so on.

The term "halogenated" or "halogen substituted" used separately or in combination herein means that one or more hydrogen atoms in an optionally substituted radical (such as alkyl, alkenyl and alkynyl) are replaced by atoms of fluorine, chlorine, bromine, iodine, or a combination thereof.

The term "aromatic base/aryl" used separately or in combination herein refers to optionally substituted aromatic hydrocarbyl having 6 to 20 cyclization carbon atoms, e.g. 6 to 12 or 6 to 10 cyclization carbon atoms, and may be a condensed aromatic ring or a non-condensed aromatic ring.

The term "heteroaryl" used separately or in combination herein refers to optionally substituted univalent heteroaryl including 5 to 20, e.g. 5 to 12, or 5 to 10 framework cyclization atoms, wherein one or more (e.g. 1 to 4, 1 to 3, or 1 to 2) cyclization atoms are heteroatoms, and the heteroatoms are independently selected from heteroatoms in oxygen, nitrogen, sulfur, phosphorus, silicon, selenium and tin, but are not limited thereby. A ring of the radical does not include two adjacent O or S atoms. Heteroaryl includes monocyclic heteroaryl or polycyclic heteroaryl (such as dicyclic heteroaryl, tricyclic heteroaryl and so on).

The term "heterocycle" or "heterocyclic radical" used separately or in combination herein refers to a non-aromatic heterocycle, including heterocycloalkyl, and heterocycloalkenyl, wherein one or more (1 to 4, 1 to 3 or 1 to 2) cyclization atoms are heteroatoms, such as oxygen atoms, nitrogen atoms, or sulfur atoms. A heterocyclic radical may include a monoheterocyclic radical (a heterocyclic radical having one ring), or a polyheterocyclic radical (e.g. a diheterocyclic radical (a heterocyclic radical having two rings), a triheterocyclic radical and so on.).

The term "carbocyclic radical" used separately or in combination herein refers to a non-aromatic carbon ring, including cycloalkyl and cycloalkenyl. The cycloalkyl may be monocyclic cycloalkyl or polycyclic cycloalkyl (e.g. having 2, 3, or 4 rings, such as dicyclic cycloalkyl), and may be a spiral ring or a bridge ring. A carbocyclic radical may have 3 to 20 carbon atoms, such as 3 to 15 cyclization carbon atoms, 3 to 10 cyclization carbon atoms or 3 to 6 cyclization carbon atoms, and may have 0, 1, 2 or 3 double bonds and/or 0, 1 or 2 triple bonds, e.g. cycloalkyl having 3 to 8 or 3 to 6 cyclization carbon atoms.

"Halogen" refers to fluorine, chlorine, bromine, and iodine, preferably fluorine, chlorine and bromine. Cyano refers to "-CN", hydroxyl refers to "-OH", mercapto refers to "-SH" and amino refers to "-NH₂".

The term "substituted" means that one or more hydrogen groups on a specific atom are substituted by a designated radical. If the normal valence of the designated atom is in, but is not beyond an existing condition, the substitution results in a stable compound.

As mentioned in the background, an omega-transaminase in the prior art still fails to satisfy demands for preparation of a compound of a chiral amine, and the present invention provides an omega-transaminase of R-configuration or a modified compound, functional equivalent, functional fragment or variant thereof in order to improve the situation above. The amino sequence of the omega-transaminase of R-configuration include a sequence selected from one of the following sequences: a) an amino acid sequence as shown in SEQ ID NO: 2; b) an amino acid sequence with at least 80% identity to the amino acid sequence as shown in SEQ ID NO: 2 and having the activity of an omega-transaminase with high stereoselective R-configuration catalytic activity, wherein the amino acid sequence is not the amino acid sequence encoded by the nucleotide sequence as shown in SEQ ID NO: 4; c) a protein which is derived from SEQ ID NO: 2 by subjecting the amino acid sequence as shown in SEQ ID NO: 2 to substitution, deletion or addition or one of more amino acids, and having the activity of an omega-transaminase with high stereoselective R-configuration catalytic activity, wherein the amino acid sequence are not the amino acid sequence encoded by the nucleotide sequence as shown in SEQ ID NO: 4, wherein the high stereoselectivity refers to the content of one of the stereoisomers being at least about 1.1 times that of the other.

The omega-transaminase of R-configuration having high stereoselectivity, or the modified compound, functional equivalent, functional fragment or variant thereof of the present invention may be used for highly efficient synthesis of a chiral amine of R-configuration with a relatively high chiral purity, and is therefore suitable for industrial production of chiral amines.

The omega-transaminase of R-configuration of the present invention refers to an omega-transaminase having high R-configuration stereoselectivity. In an embodiment, the transaminase of the present invention refers to a transaminase having a sequence as shown in SEQ ID NO: 2. The transaminase is an omega-transaminase of R-configuration acquired by subjecting a transaminase gene derived from Arthobacter sp to mutation and molecular modification by means of a molecular biological technique, wherein the nucleotide sequence of a transaminase gene taA is shown in SEQ ID NO: 4, and the corresponding amino acid sequence is shown in SEQ ID NO: 5, and the transaminase gene taA is acquired by optimizing and modifying the nucleotide sequence of the transaminase gene derived from Arthobacter sp without changing the amino acid sequence.

The amino acid sequence with at least 80% identity to the amino acid sequence as shown in SEQ ID NO: 2 and having the activity of an omega-transaminase with high stereoselective R-configuration catalytic activity refers to a sequence, which has at least 85%, 90%, 95%, 96%, 97%, 98%, 99%, 99.5% or 99.7% identity, for example, to the amino acid sequence as shown in SEQ ID NO: 2, but is not the amino acid sequence as shown in SEQ ID NO: 5. While keeping amino acid sequence playing an important role on the catalytic activity of the transaminase among the amino acid sequence as shown in SEQ ID NO: 2 unchanged, those skilled in the art may change remaining amino acid sequence of inactive sites, so that the amino acid sequence of an obtained transaminase has at least more than 80% identity to the amino acid sequence as shown in SEQ ID NO: 2, and the transaminase obtained in this way has the same transaminase activity as that of a transaminase having amino acid sequence as shown in SEQ ID NO: 2.

Similarly, one or more amino acids may be substituted, deleted or added to the amino acid sequence as shown in SEQ ID NO: 2 while keeping the amino acids playing an important role in the catalytic activity of the transaminase among the amino acid sequence as shown in SEQ ID NO: 2 unchanged, thus a protein derived from SEQ ID NO: 2 can keep the high stereoselectivity of the transaminase as shown in SEQ ID NO: 2. There may be one or more substituted, deleted or added amino acids, e.g. 1, 2, 3, 4, 5, 10, 20, 30 or 50 amino acids, e.g. substitution of conserved amino acids, wherein the amino acid sequence is not the amino acid sequence as shown in SEQ ID NO: 5. "Replacement of conserved amino acids" refers to combinations such as Gly, Ala; Val, Ile, Leu; Asp, Glu; Asn, Gln; Ser, Thr; Lys, Arg; and Phe and Tyr.

The stereoselectivity means that when two stereoisomers A and B are generated in a reaction, the yield of A is more than that of B, and the high stereoselectivity refers to the content of one of the stereoisomers being at least about 1.1 times that of the other, e.g. at least about 1.2 times, at least about 1.3 time, at least about 1.4 times, at least about 1.5 times, at least about 2 times, at least about 3 times, at least about 4 times, at least about 5 times, at least about 10 times, at least about 15 times, at least about 20 times, at least about 30 times, at least about 40 times, at least about 50 times, at least about 70 times, at least about 90 times, at least about 100 times or higher.

In the present invention, the modified compound of the omega-transaminase of R-configuration may be a chemical modified compound, such as a product of acylation, alkylation, or PEGylation, as long as these modified compounds maintain the activity of the omega-transaminase with the high stereoselectivity R-configuration catalytic activity. The functional equivalent refers to other peptide fragments that can be capable of the activity of the omega-transaminase of R-configuration. The functional fragment refers to a protein fragment that keeps the activity of the omega-transaminase with the high stereoselectivity R-configuration catalytic activity. The variant refers to a polypeptide derived from a parental protein by changing one or more amino acids at one or more (several) sites, i.e. by substation, insertion and/or deletion.

In a preferred embodiment of the present invention, the amino acid sequence of the omega-transaminase of R-configuration is an amino acid sequence acquired by substituting leucine at the 199^{th} site in the amino acid sequence as shown in SEQ ID NO: 2 by valine. The amino acid-replaced transaminase has the same activity and function as those of the transaminase as shown in SEQ ID NO: 2.

A nucleotide is provided in another typical embodiment. The nucleotide encodes the omega-transaminase of R-configuration or the modified compound, functional equivalent, functional fragment or variant thereof, and an encoding rule of the nucleotide of the omega-transaminase of R-configuration or the modified compound, functional equivalent, functional fragment or variant thereof accords with a conventional codon usage table.

In a more preferred embodiment of the present invention, the sequence of the nucleotide include a sequence selected from one of the following sequences: a) a nucleotide sequence as shown in SEO ID NO: 1; b) a nucleotide sequence with at least 80% identity to the nucleotide sequence as shown in SEQ ID NO: 1 and encoding an omega-transaminase with a high stereoselectivity R-configuration catalytic activity, wherein the nucleotide sequence is not the nucleotide sequence as shown in SEQ ID NO: 4; c) a nucleotide sequence with capable of hybridizing with the nucleotide sequence as shown in SEQ ID NO: 1 under highly stringent conditions and encoding an omega-transaminase having a high stereoselectivity R-configuration catalytic activity, wherein the nucleotide sequence is not the nucleotide sequence as shown in SEQ ID NO: 4, wherein the high stereoselectivity refers to the content of one of the stereoisomers being at least about 1.1 times that of the other.

The nucleotide sequence with at least 80% identity to the nucleotide sequence as shown in SEQ ID NO:1 and encoding the omega-transaminase having the high stereoselectivity R-configuration catalytic activity, for example, having at least 85%, 90%, 95%, 96%, 97%, 98%, 99%, 99.5%, 99.7%, 99.8% or 99.9% identity, and the nucleotide sequence is not the nucleotide sequence as shown in SEQ ID NO: 4. Based on the nucleotide sequence as shown in SEQ ID NO: 1, while keeping the nucleotides playing key roles in the catalytic activity of the transaminase unchanged, those skilled in the art may change the nucleotide sequence of remaining inactive sites so that the nucleotide sequence of an obtained transaminase has at least more than 80% identity to the nucleotide sequence as shown in SEQ ID NO: 1, and the transaminase obtained in this way has the same transaminase activity as that of a transaminase with the nucleotide sequence as shown in SEQ ID NO: 1.

The nucleotide sequence capable of hybridizing with the nucleotide sequence as shown in SEQ ID NO: 1 under highly stringent conditions and encoding the omega-transaminase having the high stereoselectivity R-configuration catalytic activity is not the nucleotide sequence as shown in SEQ ID NO: 4. Similarly, a nucleotide sequence that can be hybridized with the nucleotide sequence as shown in SEQ ID NO: 1 under highly stringent conditions and encodes the omega-transaminase having the high stereoselectivity R-configuration catalytic activity is screened based on the nucleotide sequence as shown in SEQ ID NO: 1, and a variant sequence of the nucleotide sequence as shown in SEQ ID NO: 1 is obtained in this way, having the same transaminase activity as that of the transaminase as shown in SEQ ID NO: 1.

The stereoselectivity means that when two stereoisomers A and B are generated in a reaction, the yield of A is more than that of B, and the high stereoselectivity refers to the content of one of the stereoisomers being at least about 1.1 times that of the other, e.g. at least about 1.2 times, at least about 1.3 time, at least about 1.4 times, at least about 1.5 times, at least about 2 times, at least about 3 times, at least about 4 times, at least about 5 times, at least about 10 times, at least about 15 times, at least about 20 times, at least about 30 times, at least about 40 times, at least about 50 times, at least about 70 times, at least about 90 times, at least about 100 times or higher.

An exemplary highly stringent condition may be that the hybridization is performed at 65°C by using 6X SSC and a 0.5% SDS solution, and then membrane washing is performed by 2X SSC, 0.1% SDS and 1X SSC, and 0.1 % SDS once respectively.

The term "identity" used in the present invention has a meaning generally known in the art, i.e. sequence similarity or homology. Those skilled in the art are also familiar with rules and standards for measuring the identity between different sequences. Sequences limited by the present invention with identities of different degrees also need to have the activity of the omega-transaminase with the high stereoselectivity R-configuration catalytic activity at the same time. Methods and means for screening the variant sequences by using the activity of the omega-transaminase with high stereoselective R-configuration catalytic activity are well known for those skilled in the art, and such variant sequences can be easily acquired under the teaching of the content disclosed by the present invention.

It is known for those skilled in the art that a qualifier used for defining the amino acid sequences or polynucleotides is "comprise", but it does not mean that other sequences unrelated to functions of the amino acid sequences or polynucleotides may be added randomly to both ends of the amino acid sequences or polynucleotides. It is known for those skilled in the art that it is necessary to add proper restriction sites of restriction endonuclease, or additional initiator codons or stop codons and so on to both ends of the polynucleotides so as to meet requirements of a recombination operation. Therefore, these situations cannot be truly covered if the sequences are limited by closed expression.

It is generally known for those skilled in the art that one or more codons in the nucleotide sequences may be replaced equivalently without changing the encoded amino acids. For example, the leucine Leu encoded by CTT is replaced by CTA, CTC or CTG, and the number of replaced codons may be one or more, e.g. 1, 2, 3, 4, 5, 6, 8, 9, 10, 15, 20, 30, 40 or 50, and a codon usage table is generally known in the art.

A recombinant vector is provided according to another aspect of the present invention. Any foregoing nucleotide is effectively connected in the recombinant vector. The recombinant vector of the present invention includes, but is not limited to a recombinant expression vector, and may also include a recombinant cloning vector. The recombinant vector may be a prokaryotic expression vector or a eukaryotic expression vector. In an embodiment of the present invention, the recombinant vector is a recombinant prokaryotic inducible expression vector, e.g. a series of pET vectors that induces gene expression by IPTG, such as a pET22b vector. The "effectively connected" refers to such a connection method that a polynucleotide is placed at a proper location of the vector so that the polynucleotide is copied, transcribed and/or translated correctly and smoothly. In another preferred embodiment of the present invention, the recombinant vector of the present invention is pET22b-taC including the nucleotide sequence as shown in SEQ ID NO: 1.

A host cell is provided according to another aspect of the present invention. Any foregoing recombinant vector is transformed or transfected into the host cell. The host cell of the present invention includes a prokaryotic host cell and a eukaryotic host cell. In an embodiment of the present invention, the host cell is a prokaryotic host cell, such as *Escherichia coli,* preferably, DH5α (DE3).

A method for synthesizing a chiral amine is provided according to another aspect of the present invention. The method includes the following steps: making a ketone compound, the omega-transaminase of R-configuration or the modified compound, functional equivalent, functional fragment or variant thereof, pyridoxal phosphate, and an amino donor to react in a reaction system so as to obtain the chiral amine. The method for synthesizing a chiral amine according to the present invention only needs to make appropriate adjustment to parameters including the components, proportions, use amounts, pH values, temperature and reaction time and so on of reaction raw materials of the reaction system based on a conventional method for preparing a chiral compound through a reaction catalyzed by a biological enzyme in the art.

In a preferred embodiment of the present invention, the ketone compound is 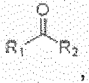 wherein R₁ and R₂ are independently C₁ to C₈ alkyl, C₅ to C₁₀ cycloalkyl, C₅ to C₁₀ aryl or C₅ to C₁₀ heteroaryl; or R₁ and R₂ form a C₅ to C₁₀ heterocyclic radical, a C₅ to C₁₀ carbocyclic radical or C₅ to C₁₀ heteroaryl with a carbon on a carbonyl group; heteroatoms in the C₅ to C₁₀ heterocyclic radical and C₅ to C₁₀ heteroaryl are independently selected from at least one of nitrogen, oxygen and sulfur; the aryl in the C₅ to C₁₀ aryl, the heteroaryl in the C₅ to C₁₀ heteroaryl, the carbocyclic radical in the C₅ to C₁₀ carbocyclic radical or the heterocyclic radical in the C₅ to C₁₀ heterocyclic radical is independently unsubstituted or is substituted by at least one radical of halogen, alkoxy or alkyl; preferably, the ketone compound 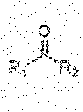 is selected from 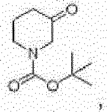 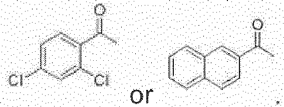 The ketone compound is a commercial raw material or a raw material that can be prepared easily and is cheap, and can therefore satisfy demands of large-scaled production.

In another preferred embodiment of the present invention, the reaction system further includes a dissolution promoter, and the dissolution promoter is dimethyl sulfoxide or polyethylene glycol, and the polyethylene glycol is preferably PEG-400. The dissolution promoter can better dissolve the raw materials above so that the reaction can be carried out, and PEG-400 has the best dissolution promoting effect.

In another preferred embodiment of the present invention, the C1 to C8 alkyl is C1 to C8 linear alkyl, the C5 to C10 heteroaryl is a pyridine group, the alkoxy is C1 to C6 alkoxy, the heterocyclic radical in the C5 to C10 heterocyclic radical is piperidine, a substituent on the aryl in the C5 to C10 aryl, the heteroaryl in the C5 to C10 heteroaryl, the carbocyclic radical in the C5 to C10 carbocyclic radical or the heterocyclic radical in the C5 to C10 heterocyclic radical is independently C1 to C6 linear alkyl or C1 to C6 alkoxy, and the amino donor is isopropylamine or D-alanine. The raw materials above are commercial raw materials or raw materials that can be prepared easily and are cheap, and can therefore satisfy demands of large-scaled production.

In a preferred embodiment of the present invention, the reaction system contains a buffer solution for maintaining the pH value of the reaction system in a range of 7.0 to 9.5, and/or wherein the ratio of the use amount of the ketone compound to that of the dissolution promoter is 1g/1mL∼15mL; and/or wherein the ratio of the use amount of the ketone compound to that of the buffer solution is 1g/15mL∼50mL, and/or wherein the ratio of the use amount of the ketone compound to that of pyridoxal phosphate is 1g/0.01g∼0.1g, and/or wherein the ratio of the use amount of the ketone compound to that of the amino donor is 1eq/1eq ∼5eq, and/or wherein the ratio of the use amount of the ketone compound to that of the omega-transaminase of R-configuration is 1g/0.2g∼10g; and/or wherein the temperature of the reaction system is 20°C-45°C and the reaction time is 12h∼48h, and/or wherein the buffer solution is a phosphate buffer solution or a triethanolamine of pH=9.3∼ 9.5.

In another preferred embodiment of the present invention, the method further includes a step that the reaction system is regulated to pH≥10 by an alkaline, and a product chiral amine in an aqueous phase is extracted by an organic solvent. Preferably, the alkaline is sodium hydroxide or potassium hydroxide, and the organic solvent is ethyl acetate, methyl tert-butyl ether or 2-methyltetrahydrofuran.

The beneficial effect of the present invention will be described below in combination with specific embodiments.

### Embodiment 1: Preparation of an omega-transaminase of R-configuration.

A method for preparing an omega-transaminase of R-configuration according to the present invention includes the following specific steps.

### (1) Template construction:

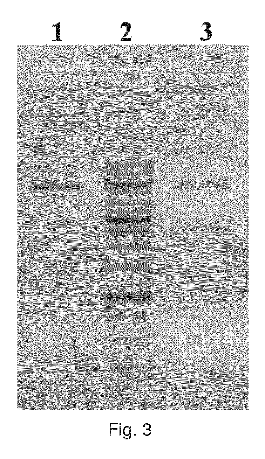

Some nucleotides of a transaminase gene TAS derived from *Arthrobacter sp.* are optimized without changing the amino acid sequence to synthesize (Sangon Biotech (Shanghai) Co., Ltd.) an optimized transaminase gene taA (sequence is shown in SEQ ID NO: 4, and the corresponding amino acid sequence is shown in SEQ ID NO: 5). The synthesized gene taA is connected to a vector pUC57 so as to obtain a recombinant vector pUC-taA. Subsequently, the recombinant vector pUC-taA and a vector pET-22b(+) are subjected to enzyme digestion simultaneously by using restriction endonuclease Nde I and Xho I. Enzyme-digested products are ligated by a T4 DNA ligase, and the ligation product is transformed into a competent cell of an DH5α strain of *Escherichia coli.* The competent cell is resuscitated by a shaker, and then coated in an LB culture dish containing ampicillin having a final concentration of 50µg/ml, and cultured overnight in an incubator at 37°C. A single colony on the culture dish is selected and inoculated in an LB liquid culture medium containing ampicillin having a final concentration of 50µg/ml, and subjected to shake at 180r/min and 37°C°C overnight. A plasmid is extracted, subjected to identification of PCR and enzyme digestion, and an identification result of the enzyme digestion is shown in Fig. 3.

Fig. 3 shows an identification diagram of the plasmid pET22b-taA through double digestion of the enzyme Nde I and the enzyme Xho I, wherein 1 represents an empty vector pET22b; 2 represents the sizes of DNA molecules (10000bp, 8000bp, 6000bp, 5000bp, 4000bp, 3500bp, 3000bp, 2500bp, 2000bp, 1500bp, 1000bp, 750bp, 500bp, 250bp respectively from top to bottom) and 3 represents pET22b-taA-DH5α. It can be seen from Fig. 3 that a relatively weak band with a fragment size of about 1000bp after the enzyme digestion is a target fragment (a plasmid band removed the target fragment is relatively strong), thus it can be determined that the insertion direction and size of an insertion sequence of the recombinant plasmid pET22b-taA are correct, and then the correct recombinant plasmid pET22b-taA is used as a template for mutation of the next step.

### (2) Cloning and expression of a mutant gene

A gene segment is designed according to a report of NCBI: 5'-ATGGCGTTTACTGTAGAAAGCCCGGCGAGTATGGATAAGGTCTTCGCC GGCTATGCTGCCCGCCAGGCTATCCTCGAAAGTACGGAGACGACGAACC CATTCGCCAAAGGTATTGCATGGGTCGAAGGTGAGTTAGTTCCGCTGGCC GAAGCACGTATCCCGATTCTCGATCAGGGCTTTTACTCCAGCGACGCGAC C-3' (SEQ ID NO:3).

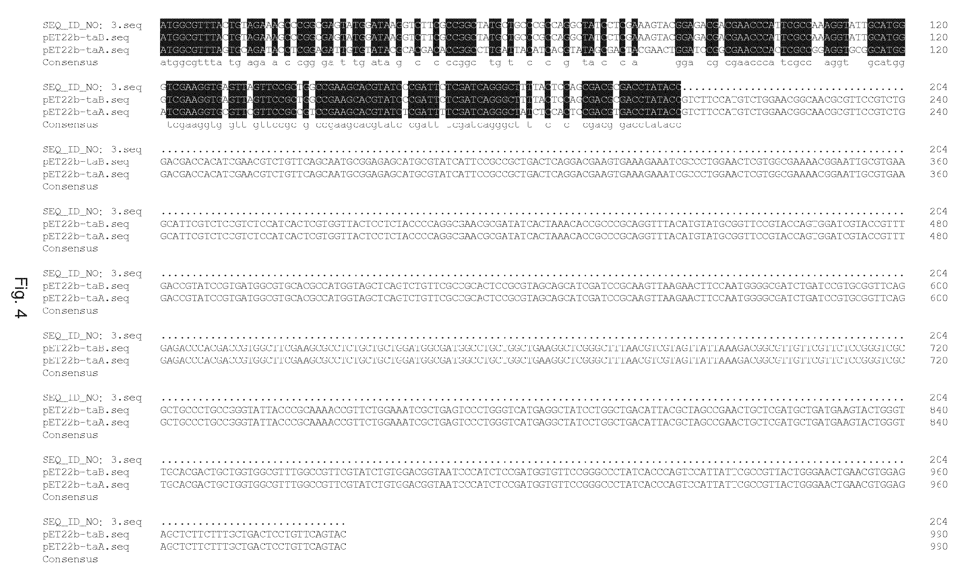

Using forward and reverse sequences of the gene above as primers, and the recombinant plasmid pET22b-taA as a template, a whole plasmid PCR is performed. After being digested by Dpn I, a PCR product is transformed into *escherichia coli* BL21 (DE3). The BL21 is resuscitated by a shaker, and then coated in an LB culture dish containing ampicillin having a final concentration of 50µg/ml, and cultured overnight at 37°C. A single colony on the culture dish is selected and inoculated in 5ml of an LB liquid culture medium containing ampicillin having a final concentration of 50µg/ml, and cultured at 180r/min and 37°C overnight. A bacterial liquid is sent to Sangon Biotech (Shanghai) Co., Ltd. to be sequenced, and a recombinant plasmid of the sequenced bacterial liquid is named BL21/pET22b-taB, the sequencing result and the primer sequence (SEQ ID NO:3) as well as a taA comparison result are shown in Fig. 4.

It can be seen from Fig. 4 that the designed sequence has completely replaced the sequence of 198bp from ATG of the taA gene carried in the L21/pET22b-taB plasmid, and the sequencing result is exactly as expected and there is no mutated base. After being identified correctly by the sequencing, the recombinant plasmid is a target plasmid sequence.

The bacterial liquid is transplanted to 5ml of an LB liquid culture medium containing ampicillin having a final concentration of 50µg/ml, subjected to shake culture at 180r/min and 37°C, and when the OD600 value is 0.6 to 0.8, IPTG is added until the final concentration is 0.2mM, the culture solution is transposed at 25°C to induce expression. Meanwhile, an IPTG inducer-free culture solution is set as negative control. After the induction is performed for 16h, the bacterial liquid is taken out and centrifuged at 12000r/min for 5min, and thalli are collected. Cells of the thalli are disrupted by an ultrasonic disruptor, and ultrasonic parameters are: probe diameter 6mm, power 200W, working time 2s, and interval 6s, totally 10min. After the ultrasonic processing, centrifugation is performed at 12000r/min for 20min at 4°C to obtain an ultrasonic supernatant and precipitate, and the supernatant is subjected to SDS-PAGE detection with a vertical electrophoresis apparatus.

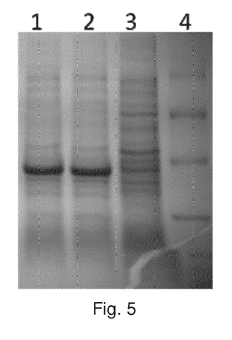

A detection result is as shown in a recombinant plasmid pET22b-taB protein expression diagram of Fig.5, where 1 represents pET22b-taB, 2 represents pET22b-taA, 3 represents pET22b and 4 represents protein makers (97.4KDa, 66.2KDa, 43KDa, 31 KDa respectively from top to bottom). It can be seen from Fig. 5 that the protein expresses well, which is not evidently different from the expression of the TaA protein and the sequencing result also shows that the inserted nucleotide sequence is correct.

### (3) Acquisition of BL21/pET22b-taC

Using the recombinant plasmid pET22b-taB as a template, a standard sequential error-prone PCR is performed (an error-prone PCR reaction system is: a 10×PCR buffer solution 10ul, dNTP 10mM 2ul, dCTP 10mM 1ul, dTTP 10mM 1ul, primers 10uM 1ul each, a template 50ng/ul 3ul, Taq DNA polymerase 0.5ul, MgCl₂ 7mM 1.4ul, MnCl₂ 0.05mM 0.2ul, H₂O 76.9ul). The primer sense strand: 5-CGCAAGTTAAGAACTTCCAATGGGGCG-3(SEQ ID NO:6) and the primer antisense strand 5-CCATTGGAAGTTCTTAACTTGCGGATCGATGC-3(SEQ ID NO:7). After being digested by Dpn I, a PCR product is transformed into *Escherichia coli* BL21 (DE3), coated in an LB culture dish containing ampicillin having a final concentration of 50µg/ml, and cultured at 37°C overnight to obtain a large number of single colonies. Single colonies in the culture dish are selected and inoculated respectively in 5ml of an LB liquid culture medium containing ampicillin having a final concentration of 50µg/ml to obtain a large amount of mutants.

Subsequently, the transaminase activity of the large amount of acquired mutants is verified according to the following steps:
the bacterial liquid of the mutants is transplanted in 100ml of an LB liquid culture medium containing ampicillin having a final concentration of 50µg/ml, subjected to shake culture at 180r/min and 37°C, and when the OD600 value is 0.6 to 0.8, IPTG is added until the final concentration is 0.2mM, and the culture solution is transposed at 25°C to induce expression. Meanwhile, an IPTG inducer-free culture solution is set as negative control. After the induction is performed for 16h, the bacterial liquid is taken out and centrifuged at 12000r/min for 5min, and thalli are collected; 0.5g of bacterial sludge is weighed and resuspended in 2.5 mL of a triethanolamine buffer solution (pH9.5), and cells of the thalli are disrupted by an ultrasonic disruptor. Ultrasonic parameters are: probe diameter 6mm, power 200W, working time 2s, and interval 6s, totally 10min. After the ultrasonic processing, centrifugation is performed at 12000r/min for 20min at 4°C to obtain an ultrasonic supernatant and precipitate, and the supernatant is used for verifying the transaminase activity in a reaction.

0.1 g of a major raw material (N-Boc-3-piperidone, CAS: 79099-07-3) and 0.1 mL of dimethyl sulfoxide are added into a reaction flask. After the raw materials are dispersed, 7.5 mL of a triethanolamine buffer solution having a concentration of 0.2mol/L and a pH value regulated to 9.3 to 9.5 by concentrated hydrochloric acid in an ice bath, 0.591g of isopropylamine, 0.0025g of pyridoxal phosphate, and 2.5mL of the large amount of mutated omega-transaminases of R-configuration are added, and stirred for 12h at a constant temperature of 30°C, wherein the pH value of the system is 9.5. The pH value of the system is regulated to above 10 by 2N NaOH, extraction is performed twice by ethyl acetate, and an organic phase is dried, filtered and concentrated to obtain a crude product (English name: (R)-1-N-Boc-3-aminopiperidine, CAS: 188111-79-7).Detected by Gas Chromatography (GC), a mutant having the best reaction is obtained through screening, having a conversion rate of 92.7% and an e.e value of 100%.

The sequence of the obtained mutant having the best transaminase activity is SEQ ID NO:1 through sequencing, and an R-configuration transaminase expressed by the mutant is named BL21/pET22b-taC.

### (4) Preparation of omega-transaminase of R-configuration

A bacterial liquid of the strain BL21/pET22b-taC is inoculated in an LB liquid culture medium containing ampicillin having a final concentration of 50µg/ml, subjected to shake culture at 180r/min and 37°C, and when the OD600 value is 0.6 to 0.8, IPTG is added until the final concentration is 0.2mM, and the culture solution is transposed at 25°C to induce expression. After the induction is performed for 16h, a fermentation solution is taken out, and centrifuged at 12000r/min for 20min, and thalli are collected. After cells of the thalli are disrupted, centrifugation is performed at 12000r/min for 20min at 4°C to obtain a supernatant, i.e. a prepared omega-transaminase of R-configuration having an amino acid sequence as shown in SEQ ID NO:2.

### Embodiment 2: Activity experiment 1 of omega-transaminase of R-configuration

1 g of a major raw material (N-Boc-4-piperidone, CAS: 79099-07-3) and 1 mL of dimethyl sulfoxide are added into a reaction flask. After the raw materials are dispersed, 50 mL of a triethanolamine buffer solution having a concentration of 0.2mol/L and a pH value regulated to 9.3 to 9.5 by concentrated hydrochloric acid in an ice bath, 0.765g of isopropylamine, 0.01g of pyridoxal phosphate, and 0.01 g of the omega-transaminase of R-configuration having the amino acid sequence as shown in SEQ ID NO:2 are added, and stirred for 12h at a constant temperature of 30°C, wherein the pH value of the system is 9.5. The pH value of the system is regulated to above 10 by 2N NaOH, extraction is performed twice by ethyl acetate, and an organic phase is dried, filtered and concentrated to obtain a crude product (English name: (R)-1-N-Boc-3-aminopiperidine, CAS: 188111-79-7). It is detected by GC that the conversion rate is 90.8% and the e.e value is 100%.

Nuclear Magnetic Resonance (NMR) data of the obtained product is as follows: 1 H-NMR (300MHz, CDCL3) δ 4.00-3.78 (m, 2H), 3.80 (m, 2H), 3.60 (m, 1 H), 1.90 (m, 1 H), 1.70 (m, 1 H), 1.60-1.40 (m, 12H), 1.30 (m, 1 H) ppm.

### Embodiment 3: Activity experiment 2 of omega-transaminase of R-configuration

0.1 g of a major raw material (2,4-dichloroacetophenone, CAS:2234-16-4) and 1.5 mL of polyethylene glycol PEG-400 are added into a reaction flask. After the raw materials are dispersed, 23.5 mL of a phosphate buffer solution (pH8.0), 0.031 g of isopropylamine, 0.0075g of pyridoxal phosphate, and 0.02g of the mutated omega-transaminase of R-configuration having the amino acid sequence as shown in SEQ ID NO:2 are added, and stirred for 20h at a constant temperature of 45°C, wherein the pH value of the system is 8.0. The pH value of the system is regulated to above 10 by 2N NaOH, extraction is performed twice by ethyl acetate, and an organic phase is dried, filtered and concentrated to obtain a crude product ([(R)-(+)-1-(2,4-dichlorophenyl)ethyl]amine). It is detected by GC that the conversion rate is 95% and the e.e value is 100%.

NMR data of the obtained product is as follows: 1 H NMR (400MHz, DMSO D6): δ=7.67 (d 1H), 7.60 (d, 1H), 7.47 (dd, 1H), 7.34 (dd, 4H), 7.23-7.12 (m, 6H), 4.84 (s, 1 H), 4.47 (quartet, 1 H), 1.31 (d, 3H).

### Embodiment 4: Activity experiment 3 of omega-transaminase of R-configuration

0.1 g of a major raw material (2-acetonaphthone, CAS: 93-08-3) and 1 mL of polyethylene glycol PEG-400 are added into a reaction flask. After the raw materials are dispersed, 24 mL of a phosphate buffer solution (pH7.0), 0.17g of isopropylamine, 0.01 g of pyridoxal phosphate, and 0.004g of the mutated omega-transaminase of R-configuration are added, and stirred for 48h at a constant temperature of 20°C, wherein the pH value of the system is 7.0. The pH value of the system is regulated to above 10 by 2N NaOH, extraction is performed twice by ethyl acetate, and an organic phase is dried, filtered and concentrated to obtain a crude product ((R)-(+)-1-(2-naphthyl)ethylamine, CAS: 3906-16-9). It is detected by GC that the conversion rate is 27% and the e.e value is 99.5%.

NMR data of the obtained product is as follows: 1 H NMR (400MHz, CDCl₃ ) δ 7.86-7.76 (m, 4H), 7.52-7.41 (m, 3H), 4.29 (q, J=6.4Hz, 1 H), 1.74 (br s, 2H), 1.48 (d, J=6.4Hz, 3H).

### Embodiment 5

The isoleucine at the 199^{th} site of the transaminase is subjected to site-directed mutagenesis based on the transaminase having the amino acid sequence as shown in SEQ ID NO:2, so as to replace the isoleucine with valine, thereby obtaining a transaminase having an amino acid sequence encoded by SEQ ID NO:8.

An enzyme activity experiment is carried out to detect the transaminase, and detection steps are as follows:
A bacterial liquid of mutants is transplanted to 100ml of an LB liquid culture medium containing ampicillin having a final concentration of 50µg/ml, subjected to shake culture at 180r/min and 37°C, and when the OD600 value is 0.6 to 0.8, IPTG is added until the final concentration is 0.2mM, and the culture solution is transposed at 25°C to induce expression. Meanwhile, an IPTG inducer-free culture solution is set as a negative control. After the induction is performed for 16h, the bacterial liquid is taken out and centrifuged at 12000r/min for 5min, and thalli are collected. 0.5g of bacterial sludge is weighed and re-suspended in 2.5 mL of a triethanolamine buffer solution (pH9.5) and cells of the thalli are disrupted by an ultrasonic disruptor. Ultrasonic parameters are: probe diameter 6mm, power 200W, working time 2s, and interval 6s, totally 10min. After the ultrasonic processing, centrifugation is performed at 12000r/min for 20min at 4°C to obtain an ultrasonic supernatant and precipitate, and the supernatant is inputted in a reaction to verify transaminase activity.

0.1g of a major raw material (N-Boc-3-piperidone, CAS: 79099-07-3) and 0.1 mL of dimethyl sulfoxide are added into a reaction flask. After the raw materials are dispersed, 7.5 mL of a triethanolamine buffer solution having a concentration of 0.2mol/L and a pH value regulated to 9.3 to 9.5 by concentrated hydrochloric acid in an ice bath, 0.591g of isopropylamine, 0.0025g of pyridoxal phosphate, and 2.5mL of the mutated omega-transaminase of R-configuration are added, and stirred for 12h at a constant temperature of 30°C, wherein the pH value of the system is 9.5. The pH value of the system is regulated to above 10 by 2N NaOH, extraction is performed twice by ethyl acetate, and an organic phase is dried, filtered and concentrated to obtain a crude product (English name: (R)-1-N-Boc-3-aminopiperidine, CAS: 188111-79-7). It is detected by GC that the conversion rate is 91.6% and the e.e value is 100%.

NMR data of the obtained product is as follows: 1H-NMR (300MHz, CDCl3) δ 4.00-3.78 (m, 2H), 3.80 (m, 2H), 3.60 (m, 1 H), 1.90 (m, 1 H), 1.70 (m, 1 H), 1.60-1.40 (m, 12H), 1.30 (m, 1 H) ppm.

It may be seen from the foregoing description that the embodiments of the present invention have achieved the following technical effect: a novel transaminase disclosed by the present invention catalyzes transfer of an amino in an amino donor to prochiral ketones or aldehydes, thereby generating corresponding chiral amines of R-configuration. The novel transaminase of the present invention is applied in preparation of a synthetic amine, which can not only convert more substrates, but also obtain an R-configuration chiral amine having a high purity stabilized at above 98%. The synthesis method, which is simple, applies easily available raw materials and has mild chemical reaction conditions, high yield and high enantiomer purity, and simple operations in the whole production process, is a feasible synthesis process with little pollution and provides a new approach and method for preparation of chiral amines.

The above are only preferred embodiments of the present invention, but are not used for limiting the present invention. For those skilled in the art, the present invention may have various modifications and changes. Any modifications, equivalent replacements, improvements and the like made within the spirit and principles of the present invention shall be included in the scope of protection of the present invention.

## Claims

**1.** An omega-transaminase of R-configuration or a modified compound, functional equivalent, functional fragment or variant thereof, wherein an amino acid sequence of the omega-transaminase of R-configuration comprises a sequence selected from one of the following sequences:
a) an amino acid sequence as shown in SEQ ID NO: 2;
b) an amino acid sequence with at least 80% identity to the amino acid sequence as shown in SEQ ID NO: 2 and having the activity of an omega-transaminase with a high stereoselectivity R-configuration catalytic activity, wherein the amino acid sequence is not an amino acid sequence encoded by a nucleotide sequence as shown in SEQ ID NO: 4;
c) a protein derived from SEQ ID NO: 2 by subjecting the amino acid sequence as shown in SEQ ID NO: 2 to substitution, deletion or addition one or more amino acids, and having the activity of an omega-transaminase with a high stereoselectivity R-configuration catalytic activity, wherein the amino acid sequence is not the amino acid sequence encoded by the nucleotide sequence as shown in SEQ ID NO: 4,
wherein the high stereoselectivity refers to the content of one of the stereoisomers being at least about 1.1 times that of the other.

**2.** The transaminase according to claim 1, wherein the amino acid sequence of the omega-transaminase of R-configuration is an amino acid sequence acquired by substituting leucine at the 199^{th} site in the amino acid sequence as shown in SEQ ID NO: 2 by valine.

**3.** A nucleotide, wherein the nucleotide encodes the omega-transaminase of R-configuration or the modified compound, functional equivalent, functional fragment or variant thereof according to claim 1 or 2.

**4.** The nucleotide according to claim 3, wherein a sequence of the nucleotide comprises a sequence selected from one of the following sequences:
a) a nucleotide sequence as shown in SEQ ID NO: 1;
b) a nucleotide sequence with at least 80% identity to the nucleotide sequence as shown in SEQ ID NO: 1 and encoding an omega-transaminase with a high stereoselectivity R-configuration catalytic activity, wherein the nucleotide sequence is not the nucleotide sequence as shown in SEQ ID NO: 4;
c) a nucleotide sequence capable of hybridizing with the nucleotide sequence as shown in SEQ ID NO: 1 under highly stringent conditions and encoding an omega-transaminase with a high stereoselectivity R-configuration catalytic activity, wherein the nucleotide sequence is not the nucleotide sequence as shown in SEQ ID NO: 4,
wherein the high stereoselectivity refers to the content of one of the stereoisomers being at least about 1.1 times that of the other.

**5.** A recombinant vector, wherein the nucleotide according to claim 3 or 4 is effectively connected in the recombinant vector.6. The recombinant vector according to claim 5, wherein the recombinant vector is pET22b-taC.7. A host cell, wherein the recombinant vector according to claim 5 or 6 is transformed or transfected in the host cell.8. A method for synthesizing a chiral amine of R configuration, wherein the method comprises the following steps: making a ketone compound, the omega-transaminase of R-configuration or the modified compound, functional equivalent, functional fragment or variant thereof according to claim 1, pyridoxal phosphate and an amino donor to react in a reaction system so as to obtain the chiral amine of R configuration.

**9.** The method according to claim 8, wherein the ketone compound is 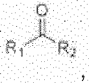 wherein R₁ and R₂ are independently C₁ to C₈ alkyl, C₅ to C₁₀ naphthenic base, C₅ to C₁₀ aryl or C₅ to C₁₀ heteroaryl; or R₁ and R₂ form a C₅ to C₁₀ heterocyclic radical, a C₅ to C₁₀ carbocyclic radical or C₅ to C₁₀ heteroaryl with a carbon on a carbonyl group; heteroatoms in the C₅ to C₁₀ heterocyclic radical and C₅ to C₁₀ heteroaryl are independently selected from at least one of nitrogen, oxygen and sulfur; the aryl in the C₅ to C₁₀ aryl, the heteroaryl in the C₅ to C₁₀ heteroaryl, the carbocyclic radical in the C₅ to C₁₀ carbocyclic radical or the heterocyclic radical in the C₅ to C₁₀ heterocyclic radical is independently unsubstituted or is substituted by at least one radical of halogen, alkoxy or alkyl; preferably, the ketone compound 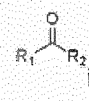 is selected from

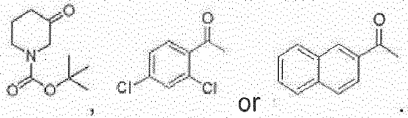

**10.** The method according to claim 8 or 9, wherein the reaction system further comprises a dissolution promoter, and the dissolution promoter is dimethyl sulfoxide or polyethylene glycol, and the polyethylene glycol is preferably PEG-400.

**11.** The method according to claim 9, wherein the C1 to C8 alkyl is C1 to C8 linear alkyl, the C5 to C10 heteroaryl is a pyridine group, the alkoxy is C1 to C6 alkoxy, the heterocyclic radical in the C5 to C10 heterocyclic radical is piperidine, a substituent on the aryl in the C5 to C10 aryl, the heteroaryl in the C5 to C10 heteroaryl, the carbocyclic radical in the C5 to C10 carbocyclic radical or the heterocyclic radical in the C5 to C10 heterocyclic radical is independently C1 to C6 linear alkyl or C1 to C6 alkoxy, and the amino donor is isopropylamine or D-alanine.
